# EUROPÄISCHE PATENTSCHRIFT

(11) **EP 4 523 526 B1**
(45) Veröffentlichungstag und Bekanntmachung des Hinweises auf die Patenterteilung: **25.03.2026**
(21) Anmeldenummer: 23197145.8
(22) Anmeldetag: 13.09.2023
(51) Int. Cl.: A01K 67/366, A01K 67/362, A01K 67/36, B65D 65/46, B65D 85/50

(54) **VERWENDUNG UND ANORDNUNG EINER VERPACKUNG FÜR VORDOSIERTE EINHEITEN ZUR INSEKTENZUCHT**
USE AND ARRANGEMENT OF A PACKAGE FOR PRE-DOSED UNITS FOR INSECT BREEDING
UTILISATION ET AGENCEMENT D'UN EMBALLAGE POUR UNITÉS PRÉDOSÉES POUR L'ÉLEVAGE D'INSECTES

(43) Veröffentlichungstag der Anmeldung: 19.03.2025
(73) Patentinhaber: Livin Farms Agrifood GmbH, 1110 Wien (AT)
(72) Erfinder: Unger, Katharina, 1120 Wien (AT); Wixler, Jürgen, 49201 Dissen am Teutoburger Wald (DE)
(74) Vertreter: Patentanwälte Pinter & Weiss OG

(56) Entgegenhaltungen:
- WO-A1-2022/232891
- US-A- 4 172 336
- US-A1- 2015 013 609

## Beschreibung

Die gegenständliche Erfindung basiert auf einer Verpackung, gefüllt mit einer vorgegebenen Menge an Insekten, wobei die Insekten in einem gleichen bestimmten Wachstumsstadium, insbesondere als Larven, oder in unterschiedlichen bestimmten Wachstumsstadien sind, wobei die Verpackung geschlossen ist, um das Austreten der Insekten aus der Verpackung zu verhindern. Die gegenständliche Erfindung betrifft eine Verwendung und eine Anordnung der Verpackung.

WO 2022/232891 A1 beschreibt einen Bausatz, bestehend aus einem biologisch abbaubaren Behälter und einer Verpackung, die Organismen wie Würmer enthält. Nach Gebrauch des Behälters wird die Verpackung geöffnet und die Organismen werden freigesetzt, um die Zersetzung des Behälters zu fördern.

Ein Lebenszyklus eines Insekts beschreibt verschiedene Wachstumsstadien (beispielsweise Ei, Larve, Puppe und erwachsenes Insekt), wobei die Dauer dieser Wachstumsstadien und die Wachstumsstadien je nach Art des Insekts variieren. Der Begriff "Insekt" umfasst dabei das Insekt in den verschiedenen Wachstumsstadien. Je nach Art der Insekten können die Wachstumsphasen einige Tage bis einige Wochen in Anspruch nehmen. In der industriellen Insektenzucht werden die Insekten insbesondere für Lebensmittel und für Tierfutter gezüchtet. Von der Produktion der Insekteneier bis hin zum Heranzüchten der erwachsenen Insekten, deckt die industrielle Insektenzucht die verschiedenen Wachstumsstadien der Insekten ab. Nachdem die Insekten als Larven aus den Eiern schlüpfen, werden diese üblicherweise in mit Substrat gefüllten Behältern oder Boxen in vorgegebene Umgebungsbedingungen gelagert, wo diese zu erwachsenen Insekten heranwachsen. Während der Lagerung in den Behältern können sich die Larven auch zur Puppe verpuppen, woraus die erwachsenen Insekten heranwachsen. Das Substrat umfasst dabei Futter für die Larven. Die erwachsenen Insekten werden "geerntet", also aus den Behältern entfernt, und können anschließend, z.B. zu proteinreichen Endprodukten wie Öl oder Pulver, weiterverarbeitet werden. Vergleichbar mit anderen Branchen werden Produktionsanlagen in der industriellen Insektenzucht ebenfalls großteils automatisiert betrieben.

In WO 2022/144197 A1, in US 2018/0070566 A1 oder in CN 115968837 A sind Layouts solcher Produktionsanlagen offenbart. Dabei werden in verschiedenen Stationen die Insekten als Larven zuerst in die mit Substrat gefüllten Behälter gefüllt, anschließend werden die Behälter eingelagert und nach einer gewissen Wachstumsperiode wieder ausgelagert und gegebenenfalls entleert (z.B., wenn die Insekten von den Larven zu erwachsenen Insekten herangewachsen sind). Die Insekten als Larven können entweder gemeinsam mit dem Substrat in die Behälter gefüllt werden oder jeweils separat. Die Behälter können dabei auch in vorgegebenen Abständen aus- und, beispielsweise nach einer Kontrolle oder nach Hinzufügen von weiterem Substrat, wieder eingelagert werden. In US 2016/0066552 A1 wird der Inhalt der Behälter mittels Kameras kontrolliert, wobei eine Dosiervorrichtung dazu dient zusätzliches Substrat in die Behälter zu füllen. Dieses Aus- und Einlagern erhöht den Aufwand während der Insektenzucht.

Für das Einfüllen der Insekten als Larven und/oder des Substrats in die Behälter sind üblicherweise Dosiervorrichtungen vorgesehen, welche eine Wiegeeinrichtung umfassen. Die Wiegeeinrichtung dient dazu, nach dem Einfüllen zu kontrollieren, ob eine vorgegebene Menge (Dosierung) an Larven und/oder Substrat im jeweiligen Behälter vorhanden ist. In WO 2022/144197 A1 oder in WO 2022/048792 A1 ist eine Dosiervorrichtung offenbart, wobei durch Wiegen überprüft wird, ob genügend Larven und/oder genügend Substrat in den Behältern vorhanden ist. Neben dem höheren Aufwand durch die Wiegeeinrichtung verlangsamt das Wiegen zudem die Produktion.

Grundsätzlich ist die Menge an Insekten als Larven vom Substrat im Behälter abhängig. Das Substrat weist dabei Eigenschaften wie Menge, Dichte, Feuchtigkeit, Nährstoffgehalt, usw., auf. Je nachdem welches Substrat mit welchen Eigenschaften im Behälter vorhanden ist, ist eine dementsprechende Menge an Larven zu dosieren, um beispielsweise eine vorgegebene Dichte der Larven (Anzahl der Larven pro Volumeneinheit) und des Substrats im Behälter zu erhalten, um einen bestmöglichen Ertrag in der Insektenzucht zu erhalten. Natürlich ist diese Dichte auch vom Behältervolumen abhängig. Die Dichte der Larven und das Substrat im Behälter beeinflusst dabei das Wachstum der Insekten. Hierfür gibt es für einen bestmöglichen Zuchterfolg einen bekannten Zusammenhang zwischen dem Substrat im Behälter und der Dichte der Insekten im Behälter.

Es ist eine Aufgabe der gegenständlichen Erfindung die Insektenzucht zu verbessern, insbesondere die Genauigkeit der Dosierung der Insekten im Behälter, beispielsweise in Abhängigkeit von einem Substrat im Behälter, zu erhöhen und somit das Wachstum der Insekten im Behälter und den Zuchterfolg zu verbessern.

Erfindungsgemäß wird die Aufgabe mit der Verwendung einer Verpackung gelöst, wobei die Verpackung dazu ausgebildet ist, sich bei zumindest einer vorgegebenen Umgebungsbedingung und/oder durch Verzehr durch die Insekten in der Verpackung zumindest teilweise zu öffnen, um die Insekten freizusetzen. Durch die Verpackung kann einerseits eine genaue Dosierung der Insekten auf ein vorgegebenes Substrat bei Verwendung in einem Behälter erzielt werden, wodurch ein optimales Wachstum der Insekten ermöglicht wird. Andererseits entfallen zusätzliche Arbeitsschritte, wie etwa das Öffnen der Verpackung.

Erfindungsgemäß ist die zumindest eine vorgegebene Umgebungsbedingung eine Temperatur und/oder eine Luftfeuchtigkeit und/oder eine Feuchtigkeit und/oder das Vorhandensein eines gasförmigen oder flüssigen Mediums am Ort der Verwendung der Verpackung. Somit ist es einfach möglich, beispielsweise bei

Verwendung der Verpackung in einem Behälter, dass sich durch das vorgegebene Substrat und dessen Eigenschaften, wie etwa eine Feuchtigkeit, die Verpackung im Behälter (selbst) zumindest teilweise öffnet und die Insekten die Verpackung durch die entstandene Öffnung verlassen können. Alternativ kann die Verpackung im Behälter auch z.B. mit Wasser besprüht werden, damit sich die Verpackung zumindest teilweise öffnet.

Vorteilhafterweise wird durch die zumindest eine vorgegebene Umgebungsbedingung eine chemische Reaktion mit einem Material der Verpackung ausgelöst, die die Verpackung zum Öffnen zumindest teilweise auflöst oder zersetzt. Dabei kann die Verpackung, beispielsweise bei Verwendung in einem Behälter, Wasser ausgesetzt werden (z.B. durch Besprühen der Verpackung), wobei das Wasser mit dem Material der Verpackung reagiert und sich die Verpackung dadurch teilweise öffnet und die Insekten in der Verpackung freigesetzt werden. Besonders vorteilhaft ist es, wenn sich die Verpackung durch die chemische Reaktion vollständig auflöst oder zersetzt, wodurch Abfall aus der Insektenzucht reduziert wird.

In einer vorteilhaften Ausführungsform ist die Verpackung zumindest teilweise luftdurchlässig, um ein Überleben der Insekten in der Verpackung, vorzugsweise für einen vorgegebenen Zeitraum, zu gewährleisten. Somit ist sichergestellt, dass das Austreten der Insekten aus der Verpackung verhindert wird, wobei die Insekten über mehrere Tage oder Wochen in der Verpackung ausreichend mit Luft versorgt sind.

In einer weiteren bevorzugten Ausführungsform ist die Verpackung luftundurchlässig, wobei eine vorgegebene Luftmenge in der Verpackung vorhanden ist, um ein Überleben der Insekten in der Verpackung, vorzugsweise für einen vorgegebene Zeitraum, zu gewährleisten. Dies ist besonders von Vorteil, denn es kann während einem Transport oder einer Lagerung der Verpackung zu einem unzulässigen Zusammendrücken der Verpackung kommen. Dadurch können die Insekten Schaden nehmen. Da jedoch die vorgegebene Luftmenge die Verpackung nicht verlassen kann, bildet die Verpackung eine Art Luftpolster, welcher das Zusammendrücken der Verpackung verhindert und so die Insekten schützt.

Vorteilhafterweise ist die Verpackung zusätzlich mit einem Substrat gefüllt, wobei das Substrat vorgegebene Eigenschaften aufweist, um ein Überleben der Insekten in der Verpackung, vorzugsweise für einen vorgegebene Zeitraum, zu gewährleisten. Dadurch kann einfach sichergestellt werden, dass die Insekten auch während eines Transports oder einer Lagerung über mehrere Tage oder Wochen ausreichend mit Feuchtigkeit, Nährstoffen, usw., versorgt sind und dadurch z.B. bis zur Verwendung in der Insektenzucht überleben.

Erfindungsgemäß wird die Verpackung in der Insektenzucht verwendet, wobei die Verpackung in einen Behälter zur Insektenzucht gegeben wird, wobei im Behälter die zumindest eine vorgegebene Umgebungsbedingung herrscht und/oder die Insekten in der Verpackung die Verpackung verzehren, um die Insekten in der Verpackung in den Behälter freizusetzen, um eine vorgegebene Dichte an Insekten im Behälter zu erhalten. Die Verpackung ermöglicht eine einfache Handhabung und Dosierung der Insekten in der Insektenzucht, wodurch das optimale Wachstum der Insekten gefördert wird.

Bei weiterer vorteilhafter Verwendung der Verpackung in der Insektenzucht wird die Verpackung zusätzlich zumindest teilweise mechanisch geöffnet, um die Insekten in der Verpackung in den Behälter freizusetzen. Dadurch kann beispielsweise zumindest eine Öffnung geschaffen werden, von der sich die Verpackung durch die zumindest eine vorgegebene Umgebungsbedingung und/oder durch den Verzehr der Insekten weiter öffnet. Somit kann sich die Zeit verkürzen bis die Insekten die Verpackung verlassen können, da bereits die zumindest eine Öffnung vorhanden ist.

Erfindungsgemäß wird die Verpackung in einem mit Substrat gefüllten Behälter zur Insektenzucht angeordnet, wobei im Behälter die zumindest eine vorgegebene Umgebungsbedingung herrscht, um die Verpackung zumindest teilweise zu öffnen und um die Larven in das Substrat freizusetzen. Somit kann die Verpackung einfach mit dem Substrat, welches beispielsweise eine vorgegebene Feuchtigkeit aufweist, in Kontakt gebracht werden, wodurch sich die Verpackung (selbst) zumindest teilweise öffnet.

Die gegenständliche Erfindung wird nachfolgend unter Bezugnahme auf die Figuren 1 bis 4 näher erläutert, die beispielhaft, schematisch und nicht einschränkend vorteilhafte Ausgestaltungen der Erfindung zeigen. Dabei zeigt
Fig. 1 den grundlegenden Aufbau der erfindungsgemäßen Verpackung,
Fig. 2 eine Anordnung der Verpackung in einem Behälter,
Fig. 3 die Verpackung im Behälter teilweise geöffnet, und
Fig. 4 die Insekten im Substrat des Behälters.

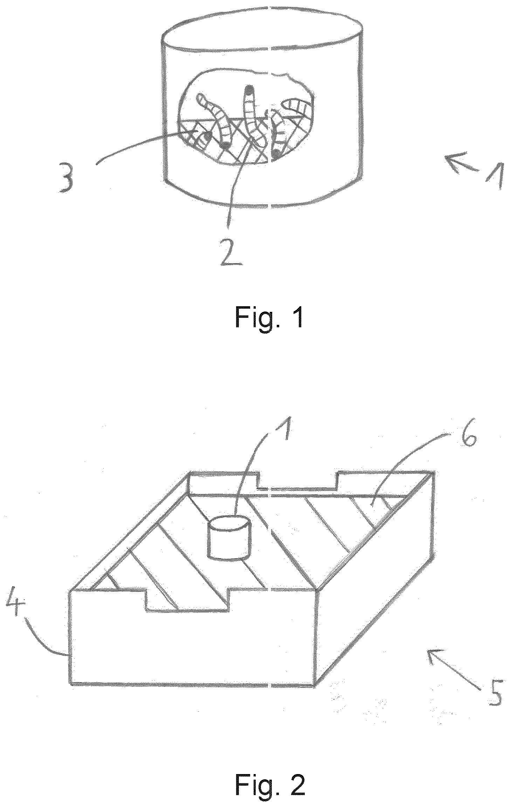

In Fig. 1 ist der grundlegende Aufbau der erfindungsgemäßen Verpackung 1 zur Insektenzucht dargestellt, wobei die Verpackung 1 lediglich zur Veranschaulichung für einen Blick in die Verpackung 1 teilweise aufgebrochen dargestellt ist.

Die Verpackung 1 ist mit einer vorgegebenen Menge an Insekten 2 gefüllt, wobei die Insekten in einem bestimmten Wachstumsstadium sind. Dabei können die Insekten 2 alle im gleichen Wachstumsstadium, insbesondere als Larven (wie in Fig. 1 dargestellt) oder als Eier, Puppen usw., sein oder in unterschiedlichen bestimmten Wachstumsstadien sein. Die Insekten 2 können dabei insbesondere jene Arten von Insekten 2 umfassen, welche zur Insektenzucht geeignet sind (z.B. Larven einer schwarzen Soldatenfliege). Die vorgegebene Menge an Insekten 2 kann beispielsweise zwischen 1g und 10g betragen.

Die Verpackung 1 ist geschlossen, um das Austreten der Insekten 2 aus der Verpackung 1 zu verhindern. Die Verpackung 1 dient damit als Transporteinheit, um eine bestimmte vorgegebene Menge an Insekten 2 in einem bestimmten Wachstumsstadium verpackt transportieren zu können. Das erleichtert die Handhabung der Insekten 2, insbesondere bei Verwendung in der Insektenzucht, erheblich.

In Fig. 1 ist die Verpackung 1 beispielhaft mit einer zylindrischen Form (z.B. ein Becher) dargestellt. Natürlich ist die Form der Verpackung 1 nicht darauf beschränkt. Insbesondere zylindrische, quadratische oder rechteckförmige Verpackungen 1 können vorteilhaft sein, um eine Mehrzahl an Verpackungen 1 platzsparend zu transportieren und/oder zu lagern. Beispiele für weitere mögliche Ausführungsformen der Verpackung 1 sind Beutel, Boxen, usw. Die Verpackung 1 kann auch unterteilt sein in Verpackungseinheiten, z.B. ein Becher mit einer Mehrzahl an Kammern in denen die Insekten 2 gefüllt sind. Die Dichte der Insekten 2 in der Verpackung 1 hängt natürlich vom Volumen der Verpackung 1 und von der Menge an Insekten 2 in der Verpackung 1 ab.

Zum Verschließen der Verpackung 1 kann ein eigener Teil der Verpackung 1 dienen, wie beispielsweise ein Deckel, Schraubverschluss usw. Ebenso ist es möglich, dass zum Verschließen kein eigener Teil vorgesehen ist, beispielsweise beim Verkleben, (Heiß)Siegeln usw. Das Verschließen der Verpackung 1 kann somit ebenfalls auf unterschiedlichste Weise erfolgen, beispielsweise durch Kleben, Siegeln, Schließen eines Deckels usw.

Die Verpackung 1 ist dazu ausgebildet, sich bei zumindest einer vorgegebenen Umgebungsbedingung und/oder durch Verzehr durch die Insekten 2 in der Verpackung 1 zumindest teilweise zu öffnen, um die Insekten 2 freizusetzen. Die Verpackung 1 an sich besteht dabei aus zumindest einem Material, wobei durch die zumindest eine vorgegebene Umgebungsbedingung beispielsweise eine chemische Reaktion mit dem zumindest einen Material der Verpackung 1 ausgelöst wird, die die Verpackung 1 zum Öffnen zumindest teilweise auflöst oder zersetzt. Die Verpackung 1 kann beispielsweise biologisch abbaubar ausgeführt sein, beispielsweise aus einem biologisch abbaubaren Kunststoff oder Papier oder Karton gefertigt sein. Die zumindest eine vorgegebene Umgebungsbedingung ist vorzugsweise eine Temperatur und/oder eine Luftfeuchtigkeit und/oder eine Feuchtigkeit und/oder das Vorhandensein eines gasförmigen oder flüssigen Mediums am Ort der Verwendung der Verpackung 1. Beispielsweise kann der Kontakt mit Wasser und/oder mit einem Substrat 6, z.B. in einem Behälter 4 (wie in den Fig. 2-4 dargestellt), zum zumindest teilweisen Abbau, Zersetzen oder Auflösen der Verpackung 1 führen, wodurch sich die Verpackung 1 zumindest teilweise öffnet und die Insekten 2 freigesetzt werden. Beispielsweise kann ein Substrat 6, in dem die Insekten 2 gezüchtet werden sollen, eine vorgegebene Feuchtigkeit oder Temperatur aufweisen, die zum zumindest teilweisen Abbau, Zersetzen oder Auflösen der Verpackung 1 führt. Welche vorgegebenen Umgebungsbedingung herrschen müssen ist vom Material der Verpackung 1 abhängig, kann aber als bekannt vorausgesetzt werden.

Weiters kann auch in der Verpackung 1 selbst die zumindest eine vorgegebene Umgebungsbedingung herrschen (z.B. Luft mit einer vorgegebenen Luftfeuchtigkeit und/oder Temperatur). Die Verpackung 1 ist dabei aber so ausgeführt, dass die Verpackung 1 die Zeit vom Abfüllen bis zur Verwendung geschlossen bleibt.

Das zumindest eine Material der Verpackung 1 kann auch dazu ausgebildet sein, um von den Insekten 2 in der Verpackung 1 verzehrt zu werden. Auch in diesem Fall ist die Verpackung 1 so ausgeführt, dass die Verpackung 1 die Zeit vom Abfüllen bis zur Verwendung geschlossen bleibt.

Beispiele für das zumindest eine Material der Verpackung 1 sind Filterpapier, Esspapier, Teigmaterial (z.B. aus Reis, Zucker, etc.), Polyvinylacetat (PVA), Kunststoff (z.B. biologisch abbaubar), usw. Dabei kann die Verpackung 1 je nach Art des Materials elastisch (verformbar) oder starr (nicht verformbar) sein. Es ist auch denkbar, dass die Verpackung 1 aus unterschiedlichen Materialien ausgeführt ist.

Jene Zeit bis sich die Verpackung 1 bei der zumindest einen vorgegebenen Umgebungsbedingung und/oder durch den Verzehr durch die Insekten 2 zumindest teilweise öffnet, ist vom Material, der Wandstärke des Materials, usw., abhängig. Beispielsweise kann so die Verpackung 1 mit unterschiedlichen Wandstärken des Materials ausgeführt sein, wodurch sich Stellen an der Verpackung 1 bilden, welche sich schneller öffnen als der Rest der Verpackung 1 (also eine Art "Sollöffnungstellen").

Die Verpackung 1 ist vor Verwendung in der Insektenzucht geschlossen, um das Austreten der Insekten 2 aus der Verpackung 1 zu verhindern. Beispielsweise kann die Verpackung 1 durch Verkleben, Verschweißen, usw. geschlossen sein, wobei die Art wie die Verpackung 1 geschlossen ist von dem verwendeten Material abhängen kann. Es ist auch denkbar die Verpackung 1, z.B. wie in Fig. 1 dargestellt als Becher, mit einem Deckel zu verschließen, welcher durch Reibung an der Verpackung 1 haftet. Natürlich kann dieser Deckel zusätzlich auch verklebt oder verschweißt sein.

Die Verpackung 1 ist vorzugsweise zumindest teilweise luftdurchlässig, um ein Überleben der Insekten 2 in der Verpackung 1, vorzugsweise für einen vorgegebenen Zeitraum, zu gewährleisten. Erstreckt sich ein Transport und/oder eine Lagerung der Verpackung 1 über mehrere Tage oder Wochen wird so sichergestellt, dass die Insekten 2 ausreichend mit Luft versorgt sind. Die Verpackung 1 kann aus diesem Grund beispielsweise eine Öffnung aufweisen, welche dazu ausgebildet sind, Luft in die Verpackung 1 durchzulassen jedoch das Austreten der Insekten 2 aus der Verpackung 1 verhindern. In einer anderen Ausführungsform ist die Verpackung 1 luftundurchlässig, wobei eine vorgegebene Luftmenge in der Verpackung 1 vorhanden ist, um ein Überleben der Insekten 2 in der Verpackung 1, vorzugsweise für einen vorgegebene Zeitraum, zu gewährleisten. Dadurch dass die Luftmenge nicht entweichen kann, bildet die Verpackung 1 eine Art Luftpolster. Somit können die Insekten 2 während dem Transport und/oder während der Lagerung der Verpackung 1, z.B. vor einem Zusammendrücken der Verpackung 1 durch äußere Einwirkungen, geschützt werden. Die vorgegebene Luftmenge in der Verpackung 1 kann beispielswiese bei der Herstellung der Verpackung 1 entstehen.

Wie in Fig. 1 dargestellt ist die Verpackung 1 vorzugsweise auch mit einem Substrat 3 gefüllt, wobei das Substrat 3 vorgegebene Eigenschaften aufweist, um ein Überleben der Insekten 2 in der Verpackung 1, vorzugsweise für einen vorgegebene Zeitraum, zu gewährleisten. Das Substrat 3 schafft für die Insekten 2 in der Verpackung 1 Bedingungen (z.B. ausreichend Feuchtigkeit, Futter, Nährstoffe, etc.), damit die Insekten 2 auch bei einem Transport und/oder einer Lagerung der Verpackung 1 über mehrere Tage oder Wochen überleben. Als Substrat 3 eignen sich dazu beispielsweise Gelatine, Agartine (Agar-Agar), Getreidemischungen oder ähnliches. Hierbei sind insbesondere die Menge der Insekten 2 in der Verpackung 1 und die Eigenschaften des Substrats 3, wie Menge, Dichte, Feuchtigkeit, Nährstoffgehalt, usw., aufeinander abgestimmt.

Grundsätzlich kann je nachdem wie die Verpackung 1 ausgestaltet ist (z.B. Material, Volumen, Eigenschaften des Substrats 3, usw.) und welche Umgebungsbedingungen vorliegen (z.B. Temperatur, Luftfeuchtigkeit, usw.) das Wachstum der Insekten 2 in der Verpackung 1 während des Transports und/oder der Lagerung beeinflusst werden. Zudem kann, beispielsweise durch einen vorgegebenen maximalen Zeitraum des Transports und/oder der Lagerung sowie durch vorgegebene Umgebungsbedingungen, verhindert werden, dass die Insekten 2 in der Verpackung 1 die Verpackung 1 vor der Verwendung in der Insektenzucht verzehren und dadurch öffnen.

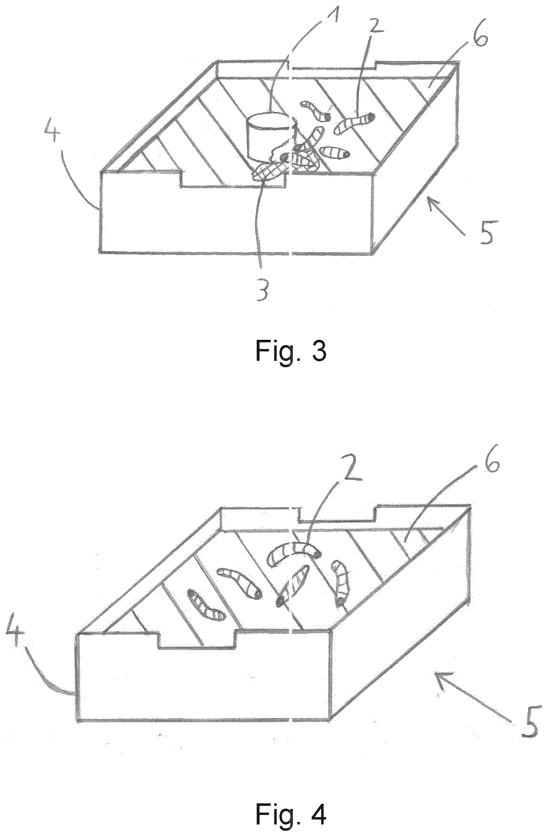

In Fig. 2 ist eine bevorzugte Anordnung 5 der Verpackung 1 in einem mit Substrat 6 gefüllten Behälter 4 zur Insektenzucht dargestellt. Im Behälter 4 herrscht dabei die zumindest eine vorgegebene Umgebungsbedingung, um die Verpackung 1 zumindest teilweise zu öffnen (wie in Fig. 3 dargestellt), damit die Insekten 2 aus der Verpackung 1 in das Substrat 6 im Behälter 4 gelangen. Die vorgegebene Umgebungsbedingung kann beispielsweise in einer Insektenzuchtanlage eingestellt werden. Das Substrat 6 im Behälter 4 kann dabei ähnliche Eigenschaften zu jenem Substrat 3 in der Verpackung 1 haben. Die zumindest eine vorgegebene Umgebungsbedingung kann beispielsweise eine Eigenschaft des Substrats 6 im Behälter 4 beschreiben, wie etwa eine Feuchtigkeit. Es ist auch möglich, dass die Verpackung 1 im Behälter 4 mit Wasser besprüht wird oder dass eine bestimmte Luftfeuchtigkeit und Temperatur eingestellt wird. Die Verpackung 1 kann bei Verwendung zusätzlich zumindest teilweise mechanisch geöffnet werden (z.B. durch Aufschneiden oder Einstanzen), um die Insekten 2 in der Verpackung 1 in den Behälter 4 freizusetzen.

In Fig. 3 ist die Verpackung 1 zumindest teilweise geöffnet dargestellt, wobei die Insekten 2 die Verpackung 1 verlassen haben und in das Substrat 6 im Behälter 4 gelangt sind. Dabei ist auch das Substrat 3 aus der Verpackung 1 in das Substrat 6 im Behälter 4 gelangt. Bleibt die Verpackung 1 noch für einen gewissen Zeitraum im Behälter 4 bestehen, kann diese dabei lokale Umgebungsbedingungen im Behälter 4 bilden (eine Art Mikroklima). Somit können die Insekten 2 noch in der Verpackung 1 bleiben, um geschützt zu sein, bis sich die Verpackung 1 vollständig aufgelöst hat und/oder von den Insekten 2 verzehrt wurde. Die lokalen Umgebungsbedingungen können für ein bessere Wachstum der Insekten 2 im Behälter 4 vorteilhaft sein, wodurch sich die Effizienz der Insektenzucht erhöht. Das Substrat 3 kann beispielsweise von den Insekten 2 verzehrt werden und/oder sich mit dem Substrat 6 im Behälter 4 vermischen.

In Fig. 4 haben sich die Insekten 2 im Substrat 6 des Behälters 4 verteilt, wobei sich die Verpackung 1 vollständig aufgelöst hat und/oder von den Insekten 2 verzehrt wurde. Somit entfällt das Entfernen der Verpackung 1 aus dem Behälter 4. Das Substrat 3 wurde beispielsweise von den Insekten 2 verzehrt und ist daher in Fig. 4 nicht mehr dargestellt. Durch die vorgegebene Menge an Insekten 2 in der Verpackung 1 liegt nun eine vorgegebene Dichte der Insekten 2 im Substrat 6 im Behälter 4 vor. Die Verpackung 1 muss sich aber nicht zwingend vollständig auflösen. Es ist durchaus möglich, dass Reste der Verpackung 1 im Behälter 4 verbleiben und diese Reste zu einem späteren Zeitpunkt entfernt werden.

## Patentansprüche

1. Verwendung einer Verpackung (1) in der Insektenzucht, indem die Verpackung (1) in einen Behälter (4) zur Insektenzucht gegeben wird, wobei die Verpackung (1) mit einer vorgegebenen Menge an Insekten (2) gefüllt ist, wobei die Insekten in einem bestimmten Wachstumsstadium, insbesondere als Larven, oder in unterschiedlichen bestimmten Wachstumsstadien sind, wobei die Verpackung (1) geschlossen ist, wodurch das Austreten der Insekten (2) aus der Verpackung (1) verhindert wird, wobei sich die Verpackung (1) bei zumindest einer vorgegebenen Umgebungsbedingung und/oder durch Verzehr durch die Insekten (2) in der Verpackung (1) zumindest teilweise öffnet, um die Insekten (2) freizusetzen, wobei die zumindest eine vorgegebene Umgebungsbedingung eine Temperatur und/oder eine Luftfeuchtigkeit und/oder eine Feuchtigkeit und/oder das Vorhandensein eines gasförmigen oder flüssigen Mediums ist, wobei im Behälter (4) die zumindest eine vorgegebene Umgebungsbedingung herrscht und/oder die Insekten (2) in der Verpackung (2) die Verpackung (1) verzehren, um die Insekten (2) in der Verpackung (1) in den Behälter (4) freizusetzen, um eine vorgegebene Dichte an Insekten (2) im Behälter (4) zu erhalten.

2. Verwendung nach Anspruch 1, **dadurch gekennzeichnet, dass** die Verpackung (1) zusätzlich zumindest teilweise mechanisch geöffnet wird, um die Insekten (2) in der Verpackung (1) in den Behälter (4) freizusetzen.

3. Verwendung nach Anspruch 1 oder 2, **dadurch gekennzeichnet, dass** die zumindest eine vorgegebene Umgebungsbedingung eine chemische Reaktion mit einem Material der Verpackung (1) auslöst, die die Verpackung (1) zum Öffnen zumindest teilweise auflöst oder zersetzt.

4. Anordnung (5) einer Verpackung (1) in einem mit Substrat (6) gefüllten Behälter (4) zur Insektenzucht, wobei die Verpackung (1) mit einer vorgegebenen Menge an Insekten (2) gefüllt ist, wobei die Insekten in einem bestimmten Wachstumsstadium, insbesondere als Larven, oder in unterschiedlichen bestimmten Wachstumsstadien sind, wobei die Verpackung (1) geschlossen ist, um das Austreten der Insekten (2) aus der Verpackung (1) zu verhindern, wobei die Verpackung (1) dazu ausgebildet ist, sich bei zumindest einer vorgegebenen Umgebungsbedingung und/oder durch Verzehr durch die Insekten (2) in der Verpackung (1) zumindest teilweise zu öffnen, um die Insekten (2) freizusetzen, wobei die zumindest eine vorgegebene Umgebungsbedingung eine Temperatur und/oder eine Luftfeuchtigkeit und/oder eine Feuchtigkeit und/oder das Vorhandensein eines gasförmigen oder flüssigen Mediums ist, wobei im Behälter (4) die zumindest eine vorgegebene Umgebungsbedingung herrscht, um die Verpackung (1) zumindest teilweise zu öffnen und um die Insekten (2) in der Verpackung (1) in das Substrat (6) im Behälter (4) freizusetzen.

5. Anordnung (5) nach Anspruch 4, **dadurch gekennzeichnet, dass** durch die zumindest eine vorgegebene Umgebungsbedingung eine chemische Reaktion mit einem Material der Verpackung (1) ausgelöst wird, die die Verpackung (1) zum Öffnen zumindest teilweise auflöst oder zersetzt.

6. Anordnung (5) nach Anspruch 4 oder 5, **dadurch gekennzeichnet, dass** die Verpackung (1) zumindest teilweise luftdurchlässig ist, um ein Überleben der Insekten (2) in der Verpackung (2), vorzugsweise für einen vorgegebenen Zeitraum, zu gewährleisten.

7. Anordnung (5) nach einem der Ansprüche 4 oder 5, **dadurch gekennzeichnet, dass** die Verpackung (1) luftundurchlässig ist, wobei eine vorgegebene Luftmenge in der Verpackung (1) vorhanden ist, um ein Überleben der Insekten (2) in der Verpackung (1), vorzugsweise für einen vorgegebene Zeitraum, zu gewährleisten.

8. Anordnung (5) nach einem der Ansprüche 4 bis 7, **dadurch gekennzeichnet, dass** die Verpackung (1) zusätzlich mit einem Substrat (3) gefüllt ist, wobei das Substrat (3) vorgegebene Eigenschaften aufweist.

## Claims

1. Use of a package (1) in insect farming, wherein the package (1) is placed in a container (4) for insect farming, wherein the package (1) is filled with a predetermined quantity of insects (2), wherein the insects are in a specific stage of growth, in particular as larvae, or in different specific stages of growth, wherein the package (1) is closed, thereby preventing the insects (2) from escaping from the package (1), wherein the package (1) opens at least partially under at least one predetermined environmental condition and/or through consumption by the insects (2) in the package (1) to release the insects (2), wherein the at least one predetermined environmental condition is a temperature and/or humidity and/or moisture and/or the presence of a gaseous or liquid medium, wherein the at least one predetermined environmental condition is present in the container (4) and/or the insects (2) consume the package (1) in order to release the insects (2) in the package (1) into the container (4) in order to obtain a predetermined density of insects (2) in the container (4).

2. Use according to claim 1, **characterized in that** the package (1) is additionally at least partially opened mechanically to release the insects (2) in the package (1) into the container (4).

3. Use according to claim 1 or 2, **characterized in that** the at least one predetermined environmental condition triggers a chemical reaction with a material of the package (1), which at least partially dissolves or decomposes the packaging (1) for opening.

4. Arrangement (5) of a package (1) in a container (4) filled with substrate (6) for insect farming, wherein the package (1) is filled with a predetermined quantity of insects (2), wherein the insects are in a specific stage of growth, in particular as larvae, or in different specific stages of growth, wherein the package (1) is closed to prevent the insects (2) from escaping from the package (1), wherein the package (1) is designed to open at least partially at at least one predetermined environmental condition and/or through consumption by the insects (2) in the package (1) to release the insects (2), wherein the at least one predetermined environmental condition is a temperature and/or humidity and/or moisture and/or the presence of a gaseous or liquid medium, wherein the at least one predetermined environmental condition is present in the container (4) in order to at least partially open the packaging (1) and to release the insects (2) in the packaging (1) into the substrate (6) in the container (4).

5. Arrangement (5) according to claim 4, **characterized in that** the at least one predetermined environmental condition triggers a chemical reaction with a material of the package (1), which dissolves or decomposes the package (1) at least partially for opening.

6. Arrangement (5) according to claim 4 or 5, **characterized in that** the package (1) is at least partially air-permeable in order to ensure the survival of the insects (2) in the package (2), preferably for a predetermined period of time.

7. Arrangement (5) according to one of claims 4 or 5, **characterized in that** the package (1) is impermeable to air, whereby a predetermined amount of air is present in the package (1) to ensure the survival of the insects (2) in the package (1), preferably for a predetermined period of time.

8. Arrangement (5) according to one of claims 4 to 7, **characterized in that** the package (1) is additionally filled with a substrate (3), wherein the substrate (3) has predetermined properties.

## Revendications

1. Utilisation d'un emballage (1) dans l'élevage d'insectes en plaçant l'emballage (1) dans un récipient (4) pour l'élevage d'insectes, dans laquelle l'emballage (1) est rempli d'une quantité prédéfinie d'insectes (2), dans laquelle les insectes sont à un stade de croissance déterminé, en particulier sous forme de larves, ou à différents stades de croissance déterminés, dans laquelle l'emballage (1) est fermé, moyennant quoi la sortie des insectes (2) hors de l'emballage (1) est empêchée, dans laquelle l'emballage (1) s'ouvre au moins partiellement dans au moins une condition ambiante prédéfinie et/ou par consommation par les insectes (2) dans l'emballage (1) afin de libérer les insectes (2), dans laquelle l'au moins une condition ambiante prédéfinie est une température et/ou une humidité de l'air et/ou une humidité et/ou la présence d'un milieu gazeux ou liquide, dans laquelle l'au moins une condition ambiante prédéfinie règne dans le récipient (4) et/ou les insectes (2) dans l'emballage (2) consomment l'emballage (1) pour libérer les insectes (2) dans l'emballage (1) dans le récipient (4) afin d'obtenir une densité prédéfinie d'insectes (2) dans le récipient (4).

2. Utilisation selon la revendication 1, **caractérisée en ce que** l'emballage (1) est en outre ouvert au moins partiellement de manière mécanique pour libérer les insectes (2) dans l'emballage (1) dans le récipient (4).

3. Utilisation selon la revendication 1 ou 2, **caractérisée en ce que** l'au moins une condition ambiante prédéfinie déclenche une réaction chimique avec un matériau de l'emballage (1) qui dissout ou décompose au moins partiellement l'emballage (1) pour l'ouvrir.

4. Agencement (5) d'un emballage (1) dans un récipient (4) rempli de substrat (6) pour l'élevage d'insectes, dans lequel l'emballage (1) est rempli d'une quantité prédéfinie d'insectes (2), dans lequel les insectes sont à un stade de croissance déterminé, en particulier sous forme de larves, ou à différents stades de croissance déterminés, dans lequel l'emballage (1) est fermé pour empêcher une sortie des insectes (2) hors de l'emballage (1), dans lequel l'emballage (1) est conçu pour s'ouvrir au moins partiellement dans au moins une condition ambiante prédéfinie et/ou par consommation par les insectes (2) dans l'emballage (1) afin de libérer les insectes (2), dans lequel l'au moins une condition ambiante prédéfinie est une température et/ou une humidité de l'air et/ou une humidité et/ou la présence d'un milieu gazeux ou liquide, dans lequel l'au moins une condition ambiante prédéfinie règne dans le récipient (4) pour ouvrir au moins partiellement l'emballage (1) et pour libérer les insectes (2) dans l'emballage (1) dans le substrat (6) dans le récipient (4).

5. Agencement (5) selon la revendication 4, **caractérisé en ce qu'**une réaction chimique avec un matériau de l'emballage (1) est déclenchée par l'au moins une condition ambiante prédéfinie, laquelle réaction chimique dissout ou décompose au moins partiellement l'emballage (1) pour l'ouvrir.

6. Agencement (5) selon la revendication 4 ou 5, **caractérisé en ce que** l'emballage (1) est au moins partiellement perméable à l'air afin d'assurer la survie des insectes (2) dans l'emballage (2), de préférence pendant une période de temps prédéfinie.

7. Agencement (5) selon l'une des revendications 4 ou 5,
**caractérisé en ce que**
l'emballage (1) est imperméable à l'air, dans lequel une quantité d'air prédéfinie est présente dans l'emballage (1) afin de garantir la survie des insectes (2) dans l'emballage (1), de préférence pendant une période de temps prédéfinie.

8. Agencement (5) selon l'une des revendications 4 à 7,
**caractérisé en ce que** l'emballage (1) est en outre rempli d'un substrat (3), dans lequel le substrat (3) présente des propriétés prédéfinies.
